# EUROPÄISCHE PATENTSCHRIFT

(11) **EP 2 681 329 B1**
(45) Veröffentlichungstag und Bekanntmachung des Hinweises auf die Patenterteilung: **22.07.2020**
(21) Anmeldenummer: 12704693.6
(22) Anmeldetag: 11.02.2012
(51) Int. Cl.: C12Q 1/28, C12Q 1/60, G01N 33/52

(54) **VERFAHREN ZUM HERSTELLEN EINES TESTELEMENTS ZUR UNTERSUCHUNG EINER KÖRPERFLÜSSIGKEITSPROBE UND TESTELEMENT**
METHOD OF PREPARING A TESTELEMENT AND TESTELEMENT FOR ANALYSING A SAMPLE OF A BODY FLUID
PROCÉDÉ POUR LA PREPARATION D'UN ELEMENT DE TEST ET ELEMENT DE TEST POUR L'EXAMEN D'UN LIQUIDE CORPOREL

(30) Priorität: 03.03.2011 EP 11001784
(43) Veröffentlichungstag der Anmeldung: 08.01.2014
(73) Patentinhaber: Roche Diabetes Care GmbH, 68305 Mannheim (DE); F. Hoffmann-La Roche AG, 4070 Basel (CH)
(72) Erfinder: FINK, Herbert, 68259 Mannheim (DE); HUELLEN, Volker, 68309 Mannheim (DE); DREIBHOLZ, Jörg, 67122 Altrip (DE)
(74) Vertreter: Twelmeier Mommer & Partner
(86) Internationale Anmeldenummer: PCT/EP2012/000653
(87) Internationale Veröffentlichungsnummer: WO 2012/116780

(56) Entgegenhaltungen:
- EP-A1- 0 254 202
- EP-A1- 0 524 596
- EP-A1- 2 284 534

## Beschreibung

Die Erfindung geht aus von einem Verfahren zur Herstellung eines Testelements zur Untersuchung einer Körperflüssigkeitsprobe mit den im Oberbegriff des Anspruchs 1 angegebenen Merkmalen, wie es aus der US 2005/0186109 A1 bekannt ist.

Herkömmliche Testelemente weisen einen Träger, üblicher Weise aus Kunststoff oder Papier, auf, der eine Nachweisschicht mit Nachweisreagenzien trägt, die bei Kontakt mit einem Analyten, beispielsweise Glucose oder Laktat, eine Nachweisreaktion bewirken. Insbesondere bei Nachweisreaktionen zur photometrischen Bestimmung einer Analytkonzentration ist eine möglichst großflächige Verteilung der Flüssigkeitsprobe auf bzw. in der Nachweisschicht vorteilhaft. Üblicher Weise wird deshalb auf die Nachweisschicht eine Spreitschicht aufgebracht, um einen aufgebrachten Flüssigkeitstropfen zu spreiten. Aus den Europäischen Patentanmeldungen EP 0524596 A1 und EP 2284534 A1 sind Testelemente mit derartigen Spreitschichten bekannt.

Für ein gutes Spreitverhalten wässriger Körperflüssigkeitsproben wie Blut muss die Spreitschicht eine hydrophile Oberfläche haben. Zudem muss die Spreitschicht eine hohe Flüssigkeitsdurchlässigkeit haben, also porös sein, damit eine Körperflüssigkeitsprobe die unter der Spreitschicht liegende Nachweisschicht gut erreichen kann.

Sehr gute Eigenschaften in dieser Hinsicht haben Spreitschichten aus Fasermaterial. Ein Nachteil von Spreitschichten aus Fasermaterialien ist allerdings, dass ein hoher Fertigungsaufwand notwendig ist, um erhebliche Schwankungen im Spreitverhalten zu vermeiden. Diese Nachteile lassen sich weitgehend vermeiden, indem als Spreitschicht eine poröse Kunststoffmembran auf die Nachweisschicht auflaminiert wird.

Aufgabe der vorliegenden Erfindung ist es, verbesserte Testelemente mit gleichmäßiger Anzeige und erhöhter Festigkeit sowie Wege zu deren Herstellung bereitzustellen.

Diese Aufgabe wir durch ein Verfahren mit den im Anspruch 1 angegebenen Merkmalen sowie durch ein Testelement gemäß Anspruch 12 gelöst. Vorteilhafte Weiterbildungen der Erfindung sind Gegenstand von Unteransprüchen.

Bei einem erfindungsgemäßen Verfahren wird die Spreitschicht auf die Nachweisschicht aufgesprüht. Diese Maßnahme hat eine Reihe von Vorteilen:
- Durch das Aufsprühen einer Membran als Spreitschicht wird die Nachweisreagenzien enthaltende Nachweisschicht wesentlich weniger mechanisch belastet als beim herkömmlichen Auflaminieren einer Kunststoffmembran oder dem Aufkleben von Fasermaterial.
- Eine aufgesprühte Spreitschicht hat eine sehr gute Haftung zu der darunter liegenden Nachweisschicht. Vorteilhaft hat ein erfindungsgemäßes Testelement deshalb eine erhöhte Verbundfestigkeit. Während Spreitschichten in Form von auflaminierten Kunststoffmembranen oder Fasermaterialien dazu neigen, sich zumindest bereichsweise von der Nachweisschicht abzulösen, lässt sich durch das Aufsprühen eine verbesserte Haftung erzielen und die Herstellung dadurch wesentlich vereinfachen. Eine aufgesprühte Spreitschicht kann beispielsweise membranartig oder vliesartig ausgebildet sein
- Durch Aufsprühen lassen sich mit geringem Aufwand Kunststoffschichten mit sehr geringen Fertigungstoleranzen herstellen. Erfindungsgemäß durch Aufsprühen hergestellte Spreitschichten haben deshalb gegenüber Spreitschichten aus Fasermaterial den Vorteil einer homogeneren Struktur. Gegenüber auflaminierten Kunststoffmembranen haben aufgesprühte Spreitschichten ebenfalls den Vorteil einer gleichbleibend besseren Qualität. Beim Auflaminieren von Kunststoffmembranen besteht nämlich stets eine mehr oder weniger große Gefahr einer Beschädigung der Membranen. Die Gefahr einer Beschädigung durch Dehnung, Rissbildung und ähnliches ist dabei umso größer, je dünner die Kunststoffmembran ist.
- Erfindungsgemäß durch Aufsprühen hergestellte Spreitschichten können in einer wesentlich geringeren Dicke als herkömmliche Spreitschichten hergestellt werden. Fasermaterial lässt sich wegen seiner Struktur nur in relativ dicken Schichten herstellen. Das Auflaminieren von Kunststoffmembranen wird bei Schichtdicken von weniger als 100 µm zunehmend problematisch und stößt bei Schichtdicken von 50 µm an seine Grenzen. Dünnere Kunststoffmembranen werden beim Auflaminieren zwangsläufig beschädigt. Durch Aufsprühen lassen sich dagegen auch Kunststoffschichten als Spreitschichten herstellen, die eine Schichtdicke von weniger als 20 µm haben, beispielsweise weniger als 12 µm, insbesondere weniger als 10 µm. Die Spreitschicht kann beispielsweise eine Dicke von 1 bis 12 µm haben, insbesondere eine Dicke von 1 bis 10 µm haben oder auch eine Dicke von 5 bis 10 µm haben. Dünnere Spreitschichten haben den Vorteil, dass eine Körperflüssigkeitsprobe die darunter liegende Nachweisschicht schneller erreichen kann. Zudem steht bei dünneren Spreitschichten ein größerer Anteil einer Probe für die eigentliche Nachweisreaktion zur Verfügung, da eine dünnere Spreitschicht einen kleineren Anteil einer Probe aufnimmt als eine dickere Spreitschicht.
- Eine dünne Spreitschicht und eine gute Haftung auf der Nachweisschicht erleichtert es, Testelemente als ein Band auszubilden, das mehrere Testfelder trägt und beispielsweise zu einer Rolle aufgewickelt sein kann. Erfindungsgemäß hergestellte Testelemente können dann mit einem dünneren Rollendurchmesser aufgewickelt werden. Als Bandkassetten ausgebildete Testelemente können dann entweder kompakter ausgebildet werden oder eine größere Anzahl von Probenuntersuchungen ermöglichen.
- Die Eigenschaften der aufgesprühten Schicht lassen sich durch Einstellen von Sprühparametern wie Luftfeuchte, Temperatur, Sprühabstand und Sprührate optimieren. Während das Ausprobieren von auflaminierten Kunststoffmembranen mit anderen Eigenschaften stets einen erheblichen Aufwand verursacht, lassen sich Sprühparameter mit vernachlässigbar geringem Aufwand variieren. Vorteilhaft können deshalb die Eigenschaften einer Spreitschicht für eine gegebene Anwendung, d.h. eine gewünschte Nachweisreaktion und die Art und Menge der Körperflüssigkeitsprobe optimiert werden.

Eine vorteilhafte Weiterbildung der Erfindung sieht vor, dass die Moleküle, welche die polymere Spreitschicht bilden, zusammen mit einer Flüssigkeit versprüht werden, die anschließend, d.h. nach dem oder beim Aufsprühen, verdunstet. Gewissermaßen wird also eine Flüssigkeit als Träger für die Moleküle verwendet, welche dann die Spreitschicht bilden. Auf diese Weise lässt sich das Aufsprühen wesentlich vereinfachen.

An sich ist es aber auch möglich, auf eine solche Trägerflüssigkeit zu verzichten, indem die Spreitschicht bildende Moleküle als Monomere oder Polymere in flüssiger Form auf die Spreitschicht aufgebracht werden. Durch Temperaturänderung oder Zugabe eines Polymerisations- bzw. Vernetzungsmittels lässt sich dann erreichen, dass sich der aufgesprühte Flüssigkeitsfilm verfestigt. Eine Polymerisation bzw. Vernetzung von Monomeren oder Polymeren lässt sich durch Temperaturänderung oder Zugabe eines Polymerisations- bzw. Vernetzungsmittels auch dann vorteilhaft verwenden, wenn Monomere bzw. Polymere zunächst mit einer Flüssigkeit, beispielsweise als Lösung oder Emulsion, auf die Nachweisschicht aufgesprüht wurden, die nach dem Aufsprühen verdunstet. Ein Polymerisations- bzw. Vernetzungsmittel kann dabei bereits in der aufgesprühten Flüssigkeit enthalten sein. Möglich ist es auch, das Polymerisations- oder Vernetzungsmittel in einem zweiten Sprühvorgang aufzubringen.

Die Moleküle, aus denen die Spreitschicht gebildet wird, können zusammen mit der Flüssigkeit, mit der sie aufgesprüht werden, eine Emulsion bilden. Bevorzug wird die Spreitschicht durch Aufsprühen einer Lösung hergestellt. Monomere oder Polymere, welche die Spreitschicht bilden, sind beim Sprühvorgang also in einer Flüssigkeit gelöst, die nach dem Aufsprühen verdunstet.

Bevorzugt ist die Flüssigkeit, mit der zusammen die Moleküle, welche die Spreitschicht bilden, aufgesprüht werden, eine organische Flüssigkeit. Besonders bevorzugt sind polare organische Lösungsmittel, insbesondere aliphatische polare organische Lösungsmittel, beispielsweise aliphatische Alkohole, Ketone und Ether oder Gemische daraus. Geeignete Flüssigkeiten sind beispielsweise Methanol, Aceton und Tetrahydrofuran.

Organische Flüssigkeiten lassen sich vorteilhaft leicht verdunsten und lösen eine Reihe von gut für die Spreitschicht geeigneten Polymeren, beispielsweise Polyethylenglykol, Polyvinylalkohol oder Cellulose einschließlich modifizierter Cellulose wie Hydroxypropylcellulose, Cellulosenitrat oder Celluloseacetat. Ein weiterer Vorteil von organischen Lösungsmitteln wie Alkohol, ist dabei dass sich auf diese Weise eine Beeinträchtigung der Nachweisschicht besonders gut vermeiden lässt. Da Körperflüssigkeitsproben wässrige Flüssigkeiten sind, enthalten die Nachweisschichten von Testelementen typischer Weise Nachweisreagenzien, die wasserlöslich sind oder zumindest sensibel auf die Anwesenheit von Wasser reagieren. Organische Flüssigkeiten wie Alkohol sind dagegen unproblematisch.

Eine weitere vorteilhafte Weiterbildung der Erfindung sieht vor, dass zum Aufsprühen eine pneumatische Düse verwendet wird. Pneumatische Düsen zerstäuben Flüssigkeiten mittels eines unter Druck stehenden Gases. Von pneumatischen Düsen wird also eine Mischung aus der zerstäubten Flüssigkeit und einem unter Druck stehenden Gas ausgestoßen. Pneumatische Düsen werden deshalb häufig auch als Zweistoffdüsen bezeichnet. Pneumatische Düsen ermöglichen eine sehr feine Zerstäubung von Flüssigkeiten und sehr gleichmäßiges Aufsprühen. Als Gas kann beispielsweise Druckluft oder Stickstoff verwendet werden.

Eine zur Ausbildung einer Spreitschicht aufgesprühte Schicht kann bei Bedarf in weiteren Fertigungsschritten bearbeitet werden. Beispielsweise kann in die Schicht ein hydrophiler Stoff eingelagert werden, beispielsweise ein Tensid, um das Spreitverhalten weiter zu verbessern. Dieser hydrophile Stoff kann bereits in der aufgesprühten Lösung oder Suspension enthalten sein und mit der Schicht mit aufgesprüht werden. Dadurch wird eine sehr homogene Verteilung in der Schicht erreicht.

Die vorliegende Erfindung betrifft ferner ein Testelement, insbesondere zur photometrischen Untersuchung einer Körperflüssigkeitsprobe, mit einem Träger, einer Nachweisreagenzien enthaltenden Nachweisschicht und einer die Nachweisschicht bedeckenden polymeren Spreitschicht, die eine Dicke von höchstens 20 µm, vorzugsweise nicht mehr als 12 µm, insbesondere nicht mehr als 10 µm, beispielsweise 1 µm bis 12 µm, insbesondere 1 µm bis 10 µm oder 5 µm bis 10 µm, hat. Mit dem erfindungsgemäßen Verfahren lassen sich Testelemente mit derartig dünnen Spreitschichten herstellen. Vergleichbare Schichtdicken lassen sich durch Auflaminieren einer herkömmlichen Membran oder Kunststofffolie nicht erreichen.

Die Spreitschicht kann zur Abtrennung von Probenbestandteilen, beispielsweise Blutkörperchen, verwendet werden. Um eine solche Abtrennung zu bewirken, sollte die Spreitschicht einen nominellen Porendurchmesser von weniger als 8 µm, besser nicht mehr als 5 µm haben. Allerdings wird durch kleinere Poren die Zeit, welche von einer Probe zum Durchdringen der Spreitschicht benötigt wird, erhöht. Vorteilhaft sind deshalb Porendurchmesser von weniger als 20 µm. Bevorzugt sind deshalb nominelle Porendurchmesser nicht mehr als 20 µm, 8 µm bis 15 µm.

Der nominelle Porendurchmesser einer Membran wird mit einer Blaspunkt-Messung gemessen. Blaspunktmessungen werden manchmal auch als Blasendruck-Tests bezeichnet.

Bevorzugt hat die Spreitschicht eine zur Nachweisschicht hin zunehmende Porengröße. Eine asymmetrische Spreitschicht lässt sich herstellen, in dem die Sprühparameter beim Aufsprühen verändert werden.

Durch Aufsprühen lassen sich sehr vorteilhaft sehr kleine, diskrete Strukturen erzeugen. Dies kann beispielsweise genutzt werden, um Testelemente auf einer Lanzette vorzusehen. Hierfür kann beispielsweise in einem ersten Schritt eine Nachweisschicht auf eine Lanzette aufgebracht, beispielsweise aufgesprüht werden. In einem weiteren Schritt kann dann eine Spreitschicht auf die Nachweisschicht aufgesprüht werden. Durch Verwendung von Mikrodüsen und/oder Masken können so winzige Testelemente in eine Lanzette integriert werden, die sich durch Auflaminieren nicht oder nur mit sehr großem Aufwand erzeugen lassen. Der Träger des Testelements kann in diesem Fall der Lanzettenkörper sein, der beispielsweise aus Metall ist.

Weitere Einzelheiten und Vorteile der Erfindung werden an Ausführungsbeispielen unter Bezugnahme auf die beigefügten Zeichnungen erläutert. Gleiche und einander entsprechende Komponenten sind dabei mit übereinstimmenden Bezugszahlen bezeichnet. Es zeigen:
- Fig. 1: eine schematische Darstellung eines Ausführungsbeispiels eines Testelements zur fotometrischen Untersuchung einer Körperflüssigkeitsprobe;
- Fig. 2: eine schematische Schnittansicht zu Figur 1; und
- Fig. 3: ein weiteres Ausführungsbeispiel eines Testelements.

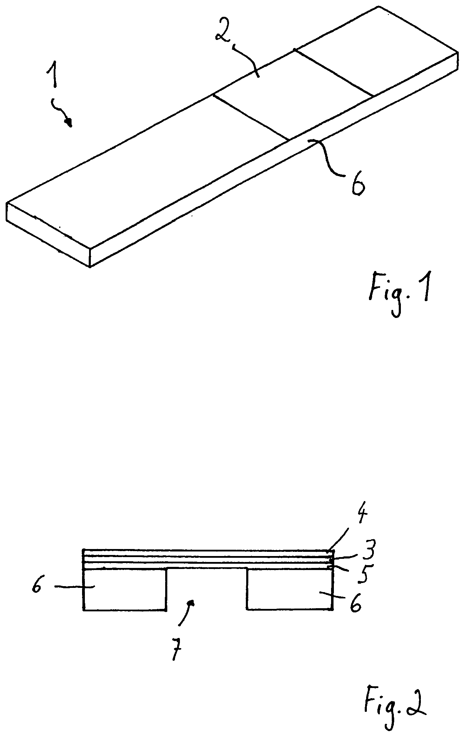

Das in Figuren 1 und 2 dargestellte Testelement 1 dient zur fotometrischen Untersuchung einer Körperflüssigkeitsprobe, beispielsweise Blut und/oder interstitieller Flüssigkeit. Mit derartigen Testelementen kann beispielsweise die Konzentration von Glukose, Lactat oder anderen medizinisch bedeutsamen Analyten bestimmt werden.

Zur fotometrischen Konzentrationsbestimmung enthält das Testelement 1 ein sogenanntes Testfeld 2. Das Testfeld hat eine Nachweisschicht 3, die Nachweisreagenzien enthält und in Figur 2 nicht maßstäblich dargestellt ist. Bei Kontakt mit den Analyten bewirken die Nachweisreagenzien eine Nachweisreaktion, die zu einer Farbänderung führt. Die Intensität der Farbänderung hängt dabei von der Analytkonzentration ab, so dass sich durch eine fotometrische Auswertung der Farbänderung die Analytkonzentration ermitteln lässt.

Die Nachweisschicht 3 ist von einer polymeren Spreitschicht 4 bedeckt. Die Spreitschicht 4 ist eine Schicht, die eine aufgebrachte Körperflüssigkeitsprobe spreitet. Durch die Spreitschicht 4 hindurch gelangt zu untersuchende Flüssigkeit dann zu der Nachweisschicht 3.

Die Nachweisschicht 3 ist auf einem Träger 5 angeordnet. Bei dem dargestellten Ausführungsbeispiel ist der Träger 5 eine transparente Kunststofffolie. Dieser Träger 5 kann auf einem zweiten Träger 6 angeordnet sein, beispielsweise einem Streifen aus Kunststoff oder Papier. Der zweite Träger 6 hat eine Aussparung 7, die von dem Träger 5 der Nachweisschicht 3 abgedeckt ist. Auf diese Weise kann eine fotometrische Messung durch die Aussparung des zweiten Trägers 6 und den transparenten ersten Träger 5 hindurch erfolgen. Die Spreitschicht 4 hat dann auf die Messung vorteilhaft keinen Einfluss.

Die Spreitschicht des in den Figuren 1 und 2 dargestellten Ausführungsbeispiels wird durch Aufsprühen einer Flüssigkeit, beispielsweise mittels einer pneumatischen Düse, auf die Nachweisschicht 3 hergestellt.

Bevorzugt werden zum Ausbilden der Spreitschicht Polymere auf die Nachweisschicht 3 aufgesprüht. Die Polymere bilden dann eine poröse Spreitschicht 4. Möglich ist es auch Monomere oder eine Mischung aus Monomeren und Polymeren auf die Nachweisschicht 3 aufzusprühen und diese dann auf der Nachweisschicht 3 zu polymerisieren. Auch Polymere können nach dem Aufsprühen vernetzt und weiter polymerisiert werden, jedoch ist dies nicht erforderlich.

Monomere oder Polymere zur Ausbildung der Spreitschicht 4 können als eine Emulsion auf die Nachweisschicht 3 aufgesprüht werden. In den Emulsionströpfchen oder beim Auftreffen auf die Nachweisschicht 3 findet dabei eine Phaseninversion statt. Die in der Emulsion enthaltenen Monomere oder Polymere können dann die Spreitschicht 4 bilden, beispielsweise als Membran oder Vlies, und sich vernetzen. Bevorzugt wird zum Ausbilden der Spreitschicht eine Lösung versprüht, insbesondere eine Polymerlösung.

Unabhängig davon ob eine Emulsion oder eine Lösung aufgesprüht wird, werden die Moleküle, welche dann die Spreitschicht 4 bilden, zusammen mit einer Flüssigkeit versprüht, die anschließend verdunstet. Diese Flüssigkeit kann eine organische Flüssigkeit sein, beispielsweise ein Alkohol. Theoretisch können auch wässrige Flüssigkeiten verwendet werden, jedoch kann die Nachweisschicht 4 durch wässrige Flüssigkeiten unter Umständen beeinträchtigt werden. Aus diesem Grund sind organische Flüssigkeiten bevorzugt.

Die Spreitschicht 4 kann beispielsweise aus Zellulose, modifizierter Zellulose, insbesondere Hydroxipropylzellulose, Zellulosenitrat oder Zelluloseacetat, Polyethylenglykol, Polysulfon, Polyethersulfon, Polyolefin, Polyurethan, Polyamid, Polyimid, Polyacrylat, Polycarbonat, Polyester, Polyether, Polyvinylether, Polyvinylester, Polyvinylalkohol, Polysiloxan oder eine Mischung, die eines oder mehrere dieser Polymere enthält, hergestellt werden. Möglich sind auch substituierte Polymere dieser Klassen, beispielsweise Polytetrafluorethylen, und Polymergemische, so dass beim Aufsprühen Copolymere entstehen.

Die Spreitschicht 4 kann vorteilhaft direkt auf die Nachweisschicht 3 aufgesprüht werden. Dies bedeutet, dass die Spreitschicht 4 die Nachweisschicht 3 kontaktiert, also an dieser anliegt. Die Spreitschicht 4 des dargestellten Ausführungsbeispiels hat eine Dicke von 5 bis 10 µm. Der geringe Abstand zwischen der Oberfläche der Spreitschicht 4 und der Nachweisschicht 3 hat den Vorteil, dass eine auf die Spreitschicht 4 aufgebrachte Körperflüssigkeitsprobe die Nachweisschicht 3 sehr schnell erreichen kann. Die Durchbruchszeit, also die Zeit bis auf die Spreitschicht 4 aufgebrachte Flüssigkeit die Nachweisschicht 3 erreicht, beträgt bei dem dargestellten Ausführungsbeispiel weniger als eine zehntel Sekunde. Die geringe Stärke der Spreitschicht 4 hat dabei auch den Vorteil, dass in der Spreitschicht 4 nur sehr wenig Flüssigkeit verbleibt und somit ein sehr großer Anteil der Probenmenge für die Nachweisreaktion in der Nachweisschicht 3 zur Verfügung steht. Für eine Konzentrationsbestimmung genügen deshalb bereits Mengen von weniger als 0,5 µl.

In die Spreitschicht 4 kann ein hydrophiler Stoff eingelagert sein, beispielsweise ein Tensid oder eine andere oberflächenaktive Substanz. Der hydrophile Stoff kann bereits zusammen mit der Spreitschicht aufgesprüht werden oder in einem nachgelagerten Fertigungsschritt in die Spreitschicht 4 eingebracht werden.

Die Spreitschicht kann vorteilhaft einen nominellen Porendurchmesser von weniger als 5 µm, vorzugsweise nicht mehr als 2 µm, besonders bevorzugt nicht mehr als 1 µm haben. Bei dem dargestellten Ausführungsbeispiel liegt der nominellen Porendurchmesser bei 0,5 µm. Auf diese Weise kann die Spreitschicht 4 zum Abfiltern von die Nachweisreaktion oder deren Auswertung störenden Probenbestandteilen benutzt werden, beispielsweise Blutkörperchen. Die Porosität der Spreitschicht 4 ist bevorzugt asymmetrisch, dies bedeutet, dass die Porengröße zur Nachweisschicht 3 hin abnimmt.

Durch Aufsprühen lassen sich Spreitschichten 4 mit einem sehr guten Spreitverhalten herstellen, beispielsweise wird bei Aufgabe von 0,5 µl Blut eine mehr als doppelt so große Fläche benetzt, wie auf einer hydrophoben Oberfläche.

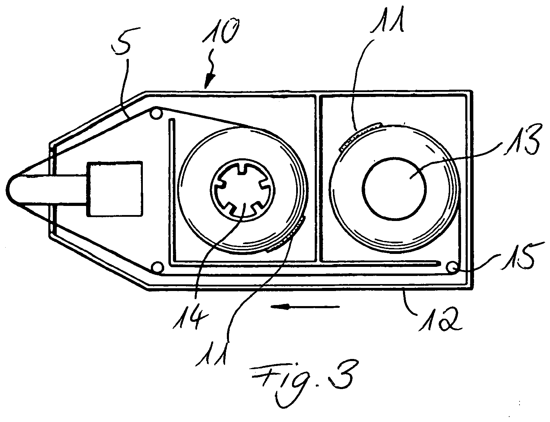

Das in den Figuren 1 und 2 dargestellte Testelement ist als ein Teststreifen ausgebildet. Figur 3 zeigt ein weiteres Ausführungsbeispiel eines Testelements zur fotometrischen Untersuchung einer Körperflüssigkeitsprobe, dass sich von dem vorstehend beschriebenen Ausführungsbeispiel im Wesentlichen nur dadurch unterscheidet, dass der Träger 5 ein Band ist, das zu einer Rolle aufgerollt in einer Bandkassette 10 angeordnet ist. Eine solche Bandkassette 10 ermöglicht es, eine sehr große Anzahl von Körperflüssigkeitsproben zu untersuchen. Die gute Haftung einer aufgesprühten Spreitschicht und deren geringe Dicke sind für derartige Bandkassetten besonders vorteilhaft, da das Aufrollen des Trägerbandes erleichtert wird.

Bei dem in Figur 3 dargestellten Ausführungsbeispiel hat der als Band ausgebildete Träger 5 mehrere Testfelder 11, die jeweils eine Nachweisschicht und eine die Nachweisschicht bedeckende Spreitschicht aufweisen, also gemäß dem Ausführungsbeispiel der Figuren 1 und 2 aufgebaut sein können. Die Bandkassette 10 hat ein Gehäuse 12, in dem eine Vorratsrolle 13 angeordnet ist, auf der der als Band ausgebildete Träger 5 mit unbenutzten Testfeldern 11 ausgewickelt ist. Gebrauchte Testfelder werden mit dem Träger 5 auf eine Antriebsrolle 14 aufgewickelt. Durch Aufwickeln der Antriebsrolle wird zugleich die Vorratsrolle abgewickelt, so dass die Testfelder 11 zum Gebrauch nacheinander zu einer Gehäuseöffnung gebracht werden können.

Die Bandkassette 10 kann eine Kammer für die Vorratsrolle 13 aufweisen. Die Antriebsrolle 14 ist bevorzugt außerhalb der Kammer der Vorratsrolle 13 angeordnet. Auf diese Weise ist die Vorratsrolle 13 mit den empfindlichen Nachweisreagenzien der unbenutzten Testfelder 11 vor schädlichen Umwelteinflüssen und insbesondere vor Feuchtigkeit in benutzten Testfeldern geschützt. Auf dem Weg von der Vorratsrolle 13 zur Antriebsrolle 14 kann der als Band ausgebildete Träger 5 von Bandführungselementen 15 geführt werden, die den Bandtransportweg vorgeben.

Effiziente Spreitschichten konnten beispielsweise auf folgende Weise erhalten werden:
Ein aliphatisches Polyurethan, beispielsweise Bionate 80A UR von DSM Biomedical Materials, Geleen, NL, wurde in einem organischen Lösungsmittel (z. B. aliphatischer Ether, etwa Tetrahydrofuran von Sigma-Aidrich, Steinheim, D) gelöst in einer Konzentration zwischen 3-6 % (m/m). Dieser Lösung wurde ein Tensid, z.B. Tween 20 (Polyoxyethylen(20)-sorbitan-monolaurat, anderer Name Poly(oxy-1,2-ethandiyl)-monododekansäure-sorbitylester, erhältlich von Sigma-Aldrich, Steinheim, D) oder DONS (Dioctylsulfosuccinat Natriumsalz, anderer Name Bis(2-ethylhexyl)-Sulfosuccinat Natriumsalz, erhältlich von Sigma-Aldrich, Steinheim, D), hinzugefügt in einer Menge entsprechend ca. 3-5 % (m/m) des Polymergehaltes. Die Lösung wurde durch eine pneumatische Drehstrahl-Düse versprüht bei einem Sprühdruck zwischen 2-4 bar. Der Verbrauch an Polymerlösung beim Sprühen lag bei ca. 2 mL/min.

Das Testelement mit der zu beschichtenden Nachweisschicht wurde in einem Abstand von 5 cm bis 15 cm, z. B. ca. 10 cm, unterhalb der Sprühdüse angeordnet. Der Prozess wurde bei Raumtemperatur in einem gängigen Laborabzug durchgeführt (ohne Kontrolle der Temperatur und Feuchte). Die aufgesprühte Polymerschicht trocknete innerhalb weniger Sekunden zu einem fest auf der Nachweisschicht anhaftenden Film.

Der Sprühvorgang wurde wahlweise mit statischem oder bewegtem Testelement durchgeführt. Zur Beschichtung mit gleichzeitiger Bewegung des Testelementes wurde das Testelement auf einem Zylinder fixiert und der Zylinder unter der Sprühdüse rotiert (z.B. mit 100-120 U/min). Sowohl die statisch als auch die mit Bewegung aufgebrachten Spreitschichten zeigten eine effiziente Spreitwirkung.

Die Schichtdicke der aufgesprühten Spreitschicht konnte einfach durch die Auswahl der Sprühzeit variiert werden. Bei einer Sprühzeit von 2 s entstanden Spreitschichten mit einer Schichtdicke im bereich von 2-5 µm. Bei einer Sprühzeit von 10 s lag die Schichtdicke der Spreitschicht im Bereich von 8-10 µm. Durch entsprechend längere Sprühzeiten konnte die Schichtdicke nach Bedarf auf Werte von z.B. 20 µm, 40 µm oder 100 µm angepasst werden.

Die Effizienz der Spreitschicht wurde überprüft durch Tüpfeln von verschiedenen Blutvolumina im Bereich 0,05 µl-2,0 µl auf die unbeschichtete Nachweisschicht bzw. auf die mit der Spreitschicht beschichtete Nachweisschicht. Bei der unbeschichteten Nachweisschicht hatte z.B. der Blutstropfen von 0,5 µl einen Durchmesser von ca. 1,5 mm, ohne auf der hydrophoben Oberfläche zu verlaufen. Es verblieb ein kalottenförmiger Blutüberstand mit einem Kontaktwinkel von ca. 40°. Bei der mit der aufgesprühten Spreitschicht beschichteten Nachweisschicht hatte der Blutstropfen von 0,5 µl einen Durchmesser von ca. 2,8 mm bis 3,1 mm und war komplett verlaufen, d.h. kein Blutüberstand mehr sichtbar (Kontaktwinkel 0°). Somit wurde durch die aufgesprühte Spreitschicht die vom Blut benetzte Fläche der Nachweisschicht in etwa vervierfacht. Die sehr effiziente Verbesserung des Spreitverhaltens wurde unabhängig von der Schichtdicke der Spreitschicht festgestellt, d.h. sowohl für dünne Spreitschichten (im Bereich von 2-5 µm) als auch für dickere Spreitschichten (im Bereich von 20-40 µm). Ebenfalls wurde eine effiziente Verbesserung des Spreitverhaltens durch die aufgesprühte Spreitschicht unabhängig vom Blutvolumen festgestellt, für Blutvolumina im relevanten Bereich von 0,05-2 µl.

Die Schichtdicke wurde durch optische Digitalmikroskopie (Auflicht) jeweils eines Querschnitts der beschichteten Testelemente bestimmt. Als Messgerät wurde ein Mikroskop Keyence VHX-1000 verwendet. In entsprechender Weise wurde auch die Effizienz der Spreitschicht bestimmt, nämlich durch optische Digitalmikroskopie im Auflicht des benetzten Bereichs eines Testelements nach Auftragen einer Blutprobe. Kontaktwinkel wurden mit einem Kontaktwinkelmessgerät Krüss DAS 100 der Firma Krüss GmbH, Hamburg bestimmt.

### Bezugszahlen

- 1: Testelement
- 2: Testfeld
- 3: Nachweisschicht
- 4: Spreitschicht
- 5: Träger
- 6: Träger
- 7: Aussparung
- 10: Bandkassette
- 11: Testfeld
- 12: Gehäuse
- 13: Vorratsrolle
- 14: Antriebsrolle
- 15: Bandführungselement

## Patentansprüche

1. Verfahren zum Herstellen eines Testelements zur Untersuchung einer Körperflüssigkeitsprobe, wobei die Nachweisschicht (3) mit einer polymeren Spreitschicht (4) bedeckt und auf einen Träger (5) aufgebracht wird, **dadurch gekennzeichnet, dass** die Spreitschicht (4) durch Aufsprühen auf die Nachweisschicht (3) hergestellt wird.

2. Verfahren nach Anspruch 1, **dadurch gekennzeichnet, dass** die Spreitschicht (4) bildende Moleküle zusammen mit einer Flüssigkeit versprüht werden, die anschließend verdunstet wird.

3. Verfahren nach Anspruch 2, **dadurch gekennzeichnet, dass** die Flüssigkeit eine organische Flüssigkeit ist.

4. Verfahren nach Anspruch 2 oder 3, **dadurch gekennzeichnet, dass** die Flüssigkeit ein polares Lösungsmittel, vorzugsweise ein aliphatisches polares organisches Lösungsmittel ist.

5. Verfahren nach einem der Ansprüche 2 bis 4, **dadurch gekennzeichnet, dass** die Flüssigkeit einen hydrophilen Stoff, vorzugsweise ein Tensid, enthält.

6. Verfahren nach einem der vorstehenden Ansprüche, **dadurch gekennzeichnet, dass** zum Herstellen der Spreitschicht (4) eine Lösung aufgesprüht wird.

7. Verfahren nach Anspruch 6, **dadurch gekennzeichnet, dass** die Lösung eine Polymerlösung ist.

8. Verfahren nach einem der vorstehenden Ansprüche, **dadurch gekennzeichnet, dass** die Spreitschicht (4) aus Polymeren hergestellt wird, die nach dem Aufsprühen vernetzen.

9. Verfahren nach einem der vorstehenden Ansprüche, **dadurch gekennzeichnet, dass** zum Aufsprühen eine pneumatische Düse verwendet wird.

10. Verfahren nach einem der vorstehenden Ansprüche, **dadurch gekennzeichnet, dass** die Spreitschicht (4) Zellulose, Polyethylenglykol, Polyvinylalkohol, Polyolefin, Polyurethan, Polyamid, Polyimid, Polyacrylat, Polycarbonat, Polyester, Polyether, Polyvinylether, Polyvinylester, Polyvinylalkohol und/oder Polysiloxan enthält.

11. Verfahren nach einem der vorstehenden Ansprüche, **dadurch gekennzeichnet, dass** die Nachweisreagenzien bei Kontakt mit einem Analyten eine Nachweisreaktion verursachen, die zu einer Farbänderung führt.

12. Testelement mit einem Träger, einer Nachweisschicht (3) und einer die Nachweisschicht (3) bedeckenden polymeren Spreitschicht (4), **dadurch gekennzeichnet, dass** die Spreitschicht (4) durch Aufsprühen auf die Nachweisschicht (3) hergestellt ist und eine Schichtdicke von weniger als 20 µm aufweist.

13. Testelement nach Anspruch 12, **dadurch gekennzeichnet, dass** die Spreitschicht (4) eine Schichtdicke von nicht mehr als 12 µm, vorzugsweise nicht mehr als 10 µm aufweist.

14. Testelement nach einem der Ansprüche 12 bis 13, **dadurch gekennzeichnet, dass** der Träger (5) ein Band ist, das zu einer Rolle aufgerollt in einer Bandkassette (10) angeordnet ist.

## Claims

1. Method for preparing a test element for analysing a body fluid sample, whereby the detection layer (3) is covered by a polymeric spreading layer (4) and is applied to a support (5), **characterised in that** the spreading layer (4) is produced by spraying it onto the detection layer (3).

2. Method according to claim 1, **characterised in that** molecules forming the spreading layer (4) are sprayed together with a liquid which subsequently evaporates.

3. Method according to claim 2, **characterised in that** the liquid is an organic liquid.

4. Method according to claim 2 or 3, **characterised in that** the liquid is a polar solvent, preferably an aliphatic polar organic solvent.

5. Method according to any one of the claims 2 to 4, **characterised in that** the liquid contains a hydrophilic substance, preferably a surfactant.

6. Method according to any one of the preceding claims, **characterised in that** a solution is applied by spraying in order to produce the spreading layer (4).

7. Method according to claim 6, **characterised in that** the solution is a polymer solution.

8. Method according to any one of the preceding claims, **characterised in that** the spreading layer (4) is produced from polymers that cross-link after being applied by spraying.

9. Method according to any one of the preceding claims, **characterised in that** a pneumatic nozzle is used for the spraying.

10. Method according to any one of the preceding claims, **characterised in that** the spreading layer (4) comprises cellulose, polyethylene glycol, polyvinylalcohol, polyolefin, polyurethane, polyamide, polyimide, polyacrylate, polycarbonate, polyester, polyether, polyvinylether, polyvinylester, polyvinylalcohol, and/or polysiloxane.

11. Method according to any one of the preceding claims, **characterised in that** the detection reagents, when exposed to an analyte, cause a detection reaction that leads to a colour change.

12. Test element comprising a support, a detection layer (3) and a polymeric spreading layer (4) covering the detection layer (3), **characterised in that** the spreading layer (4) is produced by being sprayed onto the detection layer (3).

13. Test element according to claim 12, **characterised in that** the layer thickness of the spreading layer (4) is less than 20 micrometres, preferably no more than 12 micrometres, in particular no more than 10 micrometres.

14. Test element according to any one of the claims 12 to 13, **characterised in that** the support (5) is a tape wound into a reel in a tape cartridge (10).

## Revendications

1. Procédé pour produire un élément de test pour analyser un échantillon de liquide corporel, la couche de détection (3) étant revêtue d'une couche d'étalement (4) polymère et appliquée sur un support (5), **caractérisé en ce que** la couche d'étalement (4) est produite par pulvérisation sur la couche de détection (3).

2. Procédé selon la revendication 1, **caractérisé en ce que** les molécules formant la couche d'étalement (4) sont pulvérisées conjointement avec un liquide, qui est ensuite évaporé.

3. Procédé selon la revendication 2, **caractérisé en ce que** le liquide est un liquide organique.

4. Procédé selon la revendication 2 ou 3, **caractérisé en ce que** le liquide est un solvant polaire, de préférence un solvant organique polaire aliphatique.

5. Procédé selon l'une quelconque des revendications 2 à 4, **caractérisé en ce que** le liquide contient une substance hydrophile, de préférence un tensioactif.

6. Procédé selon l'une quelconque des revendications précédentes, **caractérisé en ce que**, pour la production de la couche d'étalement (4), une solution est pulvérisée.

7. Procédé selon la revendication 6, **caractérisé en ce que** la solution est une solution polymère.

8. Procédé selon l'une quelconque des revendications précédentes, **caractérisé en ce que** la couche d'étalement (4) est produite à partir de polymères qui réticulent après la pulvérisation.

9. Procédé selon l'une quelconque des revendications précédentes, **caractérisé en ce qu'**une buse pneumatique est utilisée pour la pulvérisation.

10. Procédé selon l'une quelconque des revendications précédentes, **caractérisé en ce que** la couche d'étalement (4) contient de la cellulose, du polyéthylèneglycol, du poly(alcool vinylique), de la polyoléfine, du polyuréthane, du polyamide, du polyimide, du polyacrylate, du polycarbonate, du polyester, du polyéther, du polyvinyléther, du poly(ester de vinyle), du poly(alcool vinylique) et/ou du polysiloxane.

11. Procédé selon l'une quelconque des revendications précédentes, **caractérisé en ce que** les réactifs de détection provoquent, lors du contact avec un analyte, une réaction de détection qui entraîne une modification de couleur.

12. Élément de test présentant un support, une couche de détection (3) et une couche d'étalement (4) polymère recouvrant la couche de détection (3), **caractérisé en ce que** la couche d'étalement (4) est produite par pulvérisation sur la couche de détection (3) et présente une épaisseur de couche inférieure à 20 µm.

13. Élément de test selon la revendication 12, **caractérisé en ce que** la couche d'étalement (4) présente une épaisseur de couche inférieure ou égale à 12 µm, de préférence inférieure ou égale à 10 µm.

14. Élément de test selon l'une quelconque des revendications 12 à 13, **caractérisé en ce que** le support (5) est un ruban qui est disposé sous forme enroulée en un rouleau dans une cassette à ruban (10).
